# EUROPEAN PATENT APPLICATION

(11) **EP 4 327 851 A1**
(43) Date of publication of application: **28.02.2024**
(21) Application number: 21937921.1
(22) Date of filing: 23.04.2021
(51) Int. Cl.: A61M 15/06, A24F 40/70

(54) **METHOD FOR PRODUCING FLAVOR INHALER CARTRIDGE AND COOLING PART USED FOR FLAVOR INHALER CARTRIDGE**

(71) Applicant: Japan Tobacco Inc., Tokyo 105-6927 (JP)
(72) Inventor: TAMBO, Hitoshi, Tokyo 130-8603 (JP)
(74) Representative: Hoffmann Eitle
(86) International application number: PCT/JP2021/016427
(87) International publication number: WO 2022/224429

(57) **Abstract**

The present invention provides an innovative method for manufacturing a flavor inhaler cartridge and an innovative cooling unit used in a flavor inhaler cartridge. A method for manufacturing a flavor inhaler cartridge includes feeding a cooling unit sheet used as a material of a cooling unit, cutting the cooling unit sheet along a second direction perpendicular to a first direction in which a plurality of ridge portions extends and cutting the cooling unit sheet along the first direction so as to separate the cooling unit sheet at a predetermined interval in the second direction, spacing at least two cooling units adjacent in the first direction, which are generated from the cut cooling unit sheet, apart from each other in the first direction, and disposing the flavor source between the at least two cooling units.

## Description

### TECHNICAL FIELD

The present invention relates to a method for manufacturing a flavor inhaler cartridge and a cooling unit used in a flavor inhaler cartridge.

### BACKGROUND ART

Conventionally, there have been known flavor inhalers for inhaling a flavor or the like without burning a material. Examples of known smoking articles used in such a flavor inhaler include a smoking article including a smokable material constituted by tobacco including a volatilized component and an aerosol-cooling element that cools a volatilized material (an aerosol) before it reaches inside the user's mouth (refer to PTL 1). PTL 1 discloses a cooling element including a plurality of through holes, a cooling element including activated carbon fibers, and the like.

### CITATION LIST

### PATENT LITERATURE

PTL 1: Japanese Unexamined Patent Application Publication (Translation of PCT Application) No. 2017-518041

### SUMMARY OF INVENTION

### TECHNICAL PROBLEM

An object of the present invention is to provide an innovative method for manufacturing a flavor inhaler cartridge and an innovative cooling unit used in a flavor inhaler cartridge.

### SOLUTION TO PROBLEM

According to one aspect of the present invention, a method for manufacturing a flavor inhaler cartridge is provided. This flavor inhaler cartridge includes a flavor source configured to generate an aerosol by being heated, and a cooling unit including a corrugated portion having a plurality of ridge portions and a valley portion between the ridge portions. This manufacturing method includes feeding a cooling unit sheet used as a material of the cooling unit, cutting the cooling unit sheet along a second direction perpendicular to a first direction in which the plurality of ridge portions extends and cutting the cooling unit sheet along the first direction so as to separate the cooling unit sheet at a predetermined interval in the second direction, spacing at least two cooling units adjacent in the first direction, which are generated from the cut cooling unit sheet, apart from each other in the first direction, and disposing the flavor source between the at least two cooling units.

According to another aspect of the present invention, a cooling unit configured to be used in a flavor inhaler cartridge is provided. This cooling unit includes a corrugated portion including a plurality of ridge portions and a valley portion between the ridge portions, and a substrate sheet provided on a surface of the corrugated portion on one side.

### BRIEF DESCRIPTION OF DRAWINGS

Fig. 1A is a schematic perspective view of a flavor inhaler cartridge according to an embodiment of the present invention.
Fig. 1B is a schematic perspective view of the flavor inhaler cartridge with a case laid out.
Fig. 2 is an enlarged perspective view of a cooling unit.
Fig. 3 is a schematic view of a manufacturing apparatus for manufacturing the cartridge.
Fig. 4A is a schematic plan view of a cooling unit sheet as viewed from an arrow 4A illustrated in Fig. 3.
Fig. 4B is a schematic plan view of the cooling unit sheet immediately after it is cut by a slit cutter.
Fig. 4C is a schematic plan view of the cooling unit sheet immediately after it is cut by a cutter.
Fig. 4D is a schematic plan view of the cooling unit sheet cut on a cutting drum.
Fig. 4E is a schematic plan view illustrating two cooling units spaced apart by a separation drum and a stopper material.
Fig. 4F is a schematic plan view of the plurality of cooling units and the stopper material transferred to a conveyor.
Fig. 5A illustrates a schematic plan view of the cooling unit sheet manufactured by a manufacturing method according to another embodiment.
Fig. 5B is a schematic plan view illustrating the two cooling units spaced apart by the separation drum.
Fig. 5C is a schematic plan view of the two cooling units transferred to the conveyor.
Fig. 5D is a schematic plan view of the two cooling units transferred to the conveyor.

### DESCRIPTION OF EMBODIMENTS

In the following description, embodiments of the present invention will be described with reference to the drawings. In the drawings that will be described below, identical or corresponding components will be indicated by the same reference numerals, and redundant descriptions will be omitted.

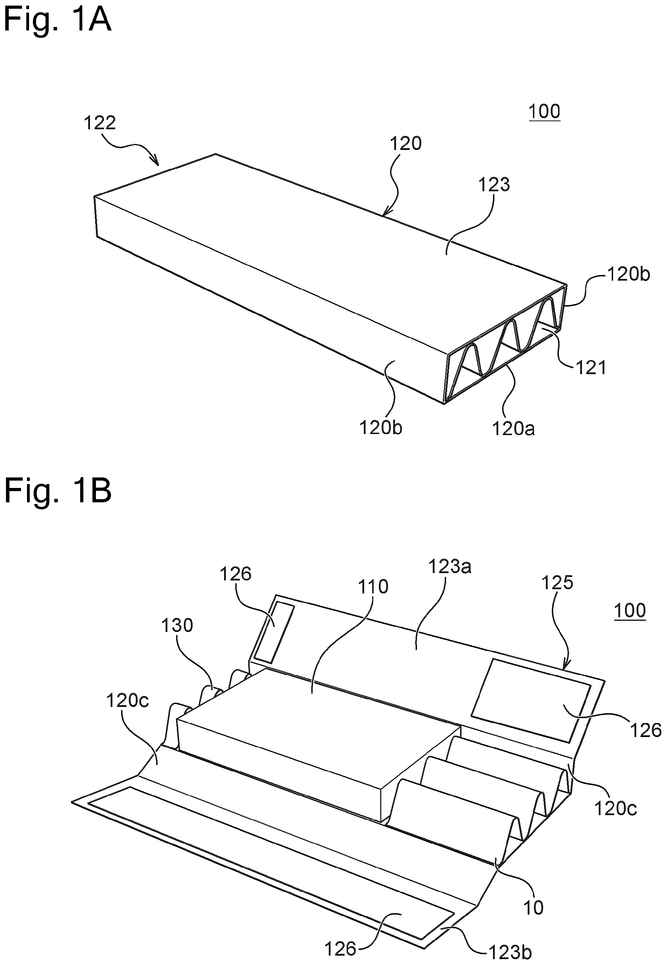

Fig. 1A is a schematic perspective view of a flavor inhaler cartridge according to the present embodiment. Fig. 1B is a schematic perspective view of the flavor inhaler cartridge with a case laid out. As illustrated in Figs. 1A and 1B, a flavor inhaler cartridge 100 according to the present embodiment includes a flavor source 110, a cooling unit 10, and a case 120. The flavor source 110 generates an aerosol by being heated. The cooling unit 10 is configured to cool the aerosol generated from the flavor source 110. Further, the cooling unit 10 also has a function of reinforcing the cartridge 100. The case 120 houses the flavor source 110 and the cooling unit 10 therein. Further, the cartridge 100 may include a stopper 130 that prevents the flavor source 110 housed in the case 120 from falling from the case 120, like the present embodiment.

As illustrated in Fig. 1A, the case 120 has a thin substantially tubular shape, and includes a first wall portion 123, a second wall 120a, and a pair of connection walls 120b. The first wall portion 123 and the second wall 120a are opposite substantially in parallel with each other. The pair of connection walls 120b connects the both ends of the first wall portion 123 and the both ends of the second wall 120a. More specifically, one of the connection walls 120b extends between one end of the first wall portion 123 and one end of the second wall 120a, and the other one of the connection walls 120b extends between the other end of the first wall portion 123 and the other end of the second wall 120a. Therefore, the substantially tubular case 120 is formed by the first wall portion 123, the second wall 120a, and the pair of connection walls 120b, and an air flow path through which the aerosol generated from the flavor source 110 passes is formed inside the case 120. The connection wall 120 illustrated in Figs. 1A and 1B is made of a single flat panel, but is not limited thereto and may be formed in an arc shape using a plurality of panels connected via embossing, debossing, or half cutting. In this case, the strength of the entire case 120 can be improved by placing the connection wall 120b in such a manner that a recessed portion formed by embossing, debossing, or half cutting faces the inside of the case 120.

Further, the case 120 includes a first opening 121 and a second opening 122 opposite from the first opening 121. The first opening 121 and the second opening 122 are defined by the first wall portion 123, the second wall 120a, and the pair of connection walls 120b. The aerosol generated from the flavor source 110 and transmitted through the cooling unit 10 can pass through the first opening 121. The first opening 121 and the second opening 122 can have substantially identical opening shapes. When the cartridge 100 is mounted on the flavor inhaler, one of the first opening 121 and the second opening 122 can be placed to face a mouthpiece side of the flavor inhaler. The first opening 121 or the second opening placed to face the mouthpiece side may be filled with a rectangular filter.

As illustrated in Fig. 1B, the case 120 can be formed by tubularly folding a single case sheet 125. The single case sheet 125 houses the flavor source 110, the cooling unit 10, and the stopper 130 therein so as to be wrapped around them. As illustrated in Fig. 1B, the single case sheet 125 includes two first walls 123a and 123b. An adhesive 126 can be applied on the respective inner surfaces of the first walls 123a and 123b. For example, a polyvinyl acetate adhesive or a CMC (carboxymethylcellulose) adhesive can be used as the adhesive 126. The adhesive 126 is not applied on a portion of the inner surface corresponding to the flavor source 110 in the first wall 123a, which is one of the first walls, so as to prevent the adhesive 126 from being attached to the flavor source 110. In other words, the inner surface of the first wall 123a is subjected to the application of the adhesive 126 so as to adhere to the cooling unit 10 and the stopper 130. Further, the adhesive 126 may be substantially entirely applied to the inner surface of the first wall 123b, which is the other of the first walls. The inner surface of the first wall 123b adheres to the outer surface of the first wall 123a adhering to the cooling unit 10 and the stopper 130. As a result, the first wall portion 123 of the case 120 is constituted by the two first walls 123a and 123b.

The thickness of the case 120 (the length between the outer surface of the first wall portion 123 and the outer surface of the second wall 120a) can be, for example, approximately 1.0 mm to approximately 5.0 mm, and can be preferably approximately 1.5 mm to approximately 3.0 mm. The length of the case 120 (the length between the first opening 121 and the second opening 122) can be, for example, approximately 15 mm to approximately 100 mm, and can be preferably approximately 30 mm to approximately 70 mm. The width of the case 120 (the length perpendicular to the thickness direction and the length direction) can be, for example, approximately 5 mm to approximately 20 mm, and can be preferably approximately 10 mm to approximately 15 mm. The case 120 can be made from, for example, predetermined thick paper. More specifically, the grammage of the paper for making the case 120 can be, for example, 50 g/m² or heavier and 200 g/m² or lighter, and can be preferably 70 g/m² or heavier and 120 g/m² or lighter. A heavier grammage of the paper for making the case 120 than 200 g/m² leads to a reduction in the heat transfer speed in a case where the cartridge 100 is directly heated, while a lighter grammage than 50 g/m² makes the case 120 easily breakable.

The shape of the flavor source 110, which is relatively easily deformable, can be maintained due to the flavor source 110 housed in the case 120. Further, making the case 120 from paper can facilitate discarding the cartridge 100 after using it and also allow a part of vapor or the aerosol generated from the flavor source 110 to be absorbed, thereby contributing to suppressing condensation of the vapor or the aerosol inside the flavor inhaler.

The flavor source 110 can include, for example, tobacco and polyhydric alcohol. The polyhydric alcohol can include glycerin, propylene glycol, sorbitol, xylitol, and erythritol. These polyhydric alcohols can be used alone or in combination of two or more thereof for the flavor source 110. More specifically, for example, the flavor source 110 can be formed by blending a binder with ground tobacco and polyhydric alcohol and carrying out tablet molding or injection molding. Examples usable as the binder include guar gum, xanthan gum, CMC (carboxymethylcellulose), CMC-Na (sodium salt of carboxymethylcellulose), pullulan or hydroxypropyl cellulose (HPC), methylcellulose, and hydroxyl methylcellulose.

The thickness of the flavor source 110 (the length corresponding to the thickness direction of the case 120) can be, for example, approximately 0.1 mm to approximately 4.5 mm, and can be preferably approximately 0.3 mm to approximately 2.5 mm. The length of the flavor source 110 (the length corresponding to the length direction of the case 120) can be, for example, approximately 10 mm to approximately 30 mm, and can be preferably approximately 15 mm to approximately 20 mm. The width of the flavor source 110 (the length corresponding to the width direction of the case 120) can be, for example, approximately 5 mm to approximately 20 mm, and can be preferably approximately 10 mm to approximately 15 mm. Examples employable as the flavor source 110 include a molded body manufactured by extrusion molding or tablet molding, and a tobacco sheet formed by a sheet-forming method, a casting method, or a rolling method or an article formed by folding this tobacco sheet.

A groove may be formed on a surface of the flavor source 110 that faces the first wall portion 123 or a surface of the flavor source 110 that faces the second wall 120a along a direction extending between the first opening 121 and the second opening 122 of the case 120 (hereinafter referred to as a length direction). This arrangement can make it easy for the aerosol generated from the flavor source 110 to move to the first opening 121 by passing through the groove on the surface of the flavor source 110. In other words, this configuration allows the groove on the surface of the flavor source 110 to function as an aerosol flow path.

As illustrated in Fig. 1B, the flavor source 110 and the cooling unit 10 can be arranged adjacent in the length direction. Further, the stopper 130 can be arranged adjacent to the flavor source 110 opposite from the cooling unit 10. In other words, the flavor source 110 is interposed between the stopper 130 and the cooling unit 10 in the length direction.

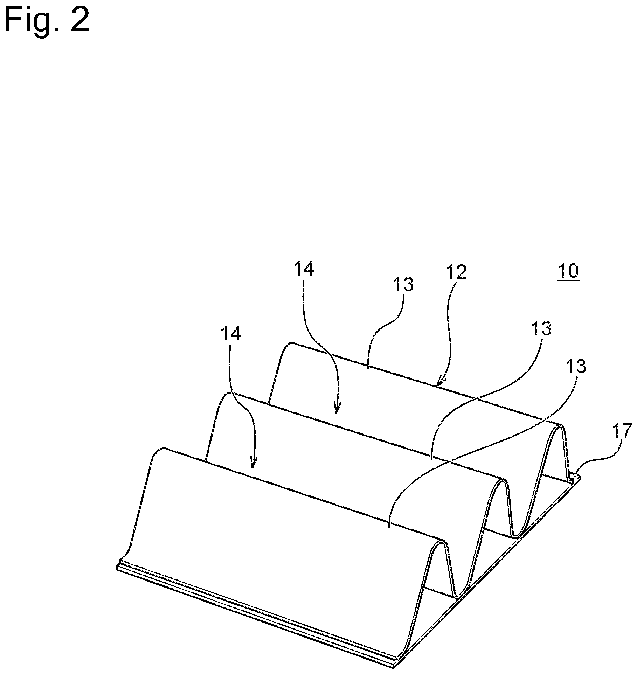

Fig. 2 is an enlarged perspective view of the cooling unit 10. As illustrated in Fig. 2, the cooling unit 10 includes a corrugated portion 12 having a plurality of ridge portions 13 and valley portions 14 between the ridge portions 13. As will be used herein, the direction in which the plurality of ridge portions 13 extends will be referred to as a first direction, and the direction in which the plurality of ridge portions 13 is adjacent, i.e., the direction perpendicular to the first direction will be referred to as a second direction. The corrugated portion 12 illustrated in Fig. 2 has a substantially sinusoidal shape as viewed from the first direction, but is not limited thereto and may have a square-wave shape or a triangle-wave shape. The corrugated portion 12 can be made from, for example, one or more materials selected from the group consisting of paper, polyethylene, polypropylene, polyvinyl chloride, polyethylene terephthalate, polylactic acid, cellulose acetate, and aluminum foil.

The cooling unit 10 can further include a substrate sheet 17 provided on a surface of the corrugated portion 12 on any one side, i.e., a protrusion-side surface defined by the ridge portions 13 or a recessed-side surface defined by the valley portions 14. In the present embodiment illustrated in Fig. 2, the substrate sheet 17 is provided on the recessed-side surface defined by the valley portions 14. The substrate sheet 17 may be provided on each of the both surfaces of the corrugated portion 12. The substrate sheet 17 can be made from, for example, one or more materials selected from the group consisting of paper, polyethylene, polypropylene, polyvinyl chloride, polyethylene terephthalate, polylactic acid, cellulose acetate, and aluminum foil. In the case where the substrate sheet 17 is provided on only one side of the corrugated portion 12, the substrate sheet 17 is preferably provided on a surface of the corrugated portion 12 that faces the second wall 120a in a state that the cooling unit 10 is housed in the case 120.

As illustrated in Fig. 1B, the cooling unit 10 is housed in the case 120 while being oriented in such a manner that the first direction in which the ridge portions 13 of the corrugated portion 12 extend matches the length direction of the cartridge 100. Due to that, the aerosol generated in the flavor source 110 can pass through spaces between the ridge portions 13 and spaces between the valley portions 14 of the corrugated portion 12. In other words, the spaces between the ridge portions 13 and the spaces between the valley portions 14 of the corrugated portion 12 can function as an aerosol flow path.

As illustrated in Fig. 1B, in the present embodiment, the stopper 130 can be structured similarly to the cooling unit 10. However, without being limited thereto, the stopper 130 may be made from any material that allows air introduced from the second opening 122 to pass therethrough and allows the flavor source 110 to be prevented from falling from the case 120. Further, the stopper 130 may be omitted from the cartridge 100.

The cartridge 100 illustrated in Fig. 1A generates the vapor and the aerosol of the aerosol source or the flavor source 110 by being heated by a heating unit of the flavor inhaler. The vapor and the aerosol generated in the flavor source 110 of the cartridge 100 are cooled by passing through the cooling unit 10 and reach inside the user's mouth by being drawn by the user. The vapor generated in the cartridge 100 can be atomized into an aerosol by being cooled by the cooling unit 10. In the present embodiment, the cartridge 100 is provided in the form of a thin plate or a card.

The cartridge 100 illustrated in Fig. 1A can be heated by the heating unit of the flavor inhaler from one side that is the first wall portion 123 or the second wall 120a of the case 120. In this case, heat is gradually transferred from the one surface side of the flavor source 110 and the smoking time can be increased. Alternatively, the cartridge 100 may be heated by the heating unit of the flavor inhaler from the both sides that are the first wall portion 123 and the second wall 120a of the case 120. Further, the flavor inhaler may be configured to include a susceptor in the cartridge 100, thereby inductively heating the susceptor using an induction coil, and heating the flavor source 110 by that. The cartridge 100 can be heated to, for example, 200°C or higher and 300°C or lower.

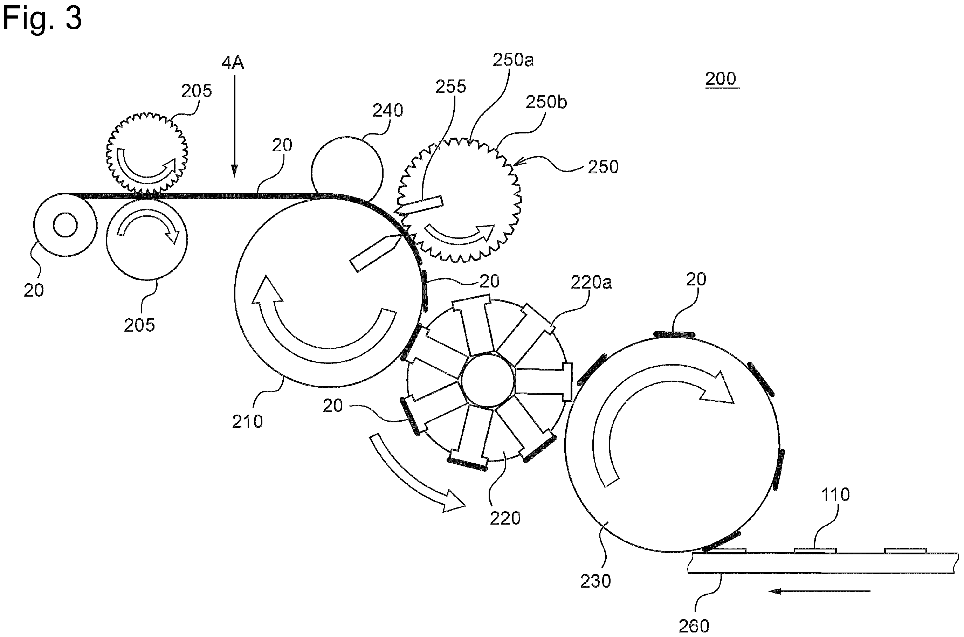

Next, a method for manufacturing the cartridge 100 illustrated in Figs. 1A and 1B will be described. Fig. 3 is a schematic view of a manufacturing apparatus for manufacturing the cartridge 100. In Fig. 3, rotational directions of drums and rollers are indicated by arrows. More specifically, an apparatus 200 illustrated in Fig. 3 is an apparatus that supplies the cooling unit 10 to the flavor source 110. The apparatus 200 illustrated in Fig. 3 manufactures the cooling unit 10 illustrated in Figs. 1B and 2 by appropriately cutting a cooling unit sheet 20 used as a material of the cooling unit 10 of the cartridge 100 while conveying it by a plurality of drums. Further, the apparatus 200 illustrated in Fig. 3 places the manufactured cooling unit 10 adjacent to the flavor source 110 as illustrated in Fig. 1B.

The apparatus 200 includes a pair of first feed rollers 205, a cutting drum 210, a second feed roller 250, an acceleration drum 220, and a separation drum 230 (a separating drum). The cutting drum 210 conveys the cooling unit sheet 20 while attracting it, and the cooling unit sheet 20 is cut on the cutting drum 210. The acceleration drum 220 receives the cut cooling unit sheet 20 from the cutting drum 210. The acceleration drum 220 rotates in an opposite direction from the cutting drum 210. The separation drum 230 spaces the cooling unit 10 apart in the width direction of the separation drum 230 (the depth direction of the paper of Fig. 3) on the cut cooling unit sheet 20. The separation drum 230 rotates in the same direction as the cutting drum 210. The cartridge 100 is manufactured using the cutting drum 210, the acceleration drum 220, and the separation drum 230 in the present embodiment, but the number of drums is not limited thereto. Separating the cooling unit sheet 20 using the single separation drum 230 may lead to an increase in the diameter of the separation drum 230 or facilitate detachment of the cooling unit sheet 20 due to an increase in the separation speed. Therefore, in the case where the cooling unit sheet 20 is separated, the cooling unit sheet 20 is preferably separated in a stepwise manner by providing two separation drums 230.

Preferably, the cooling unit sheet 20 is wound into a roll. This arrangement allows the cooling unit sheet 20 to be disposed in a smaller space and the cooling unit 10 to be continuously manufactured on the apparatus 200. Further, preferably, the cooling unit sheet 20 is wound in such a manner that the corrugated portion 12 faces outward and the substrate sheet 17 faces inward.

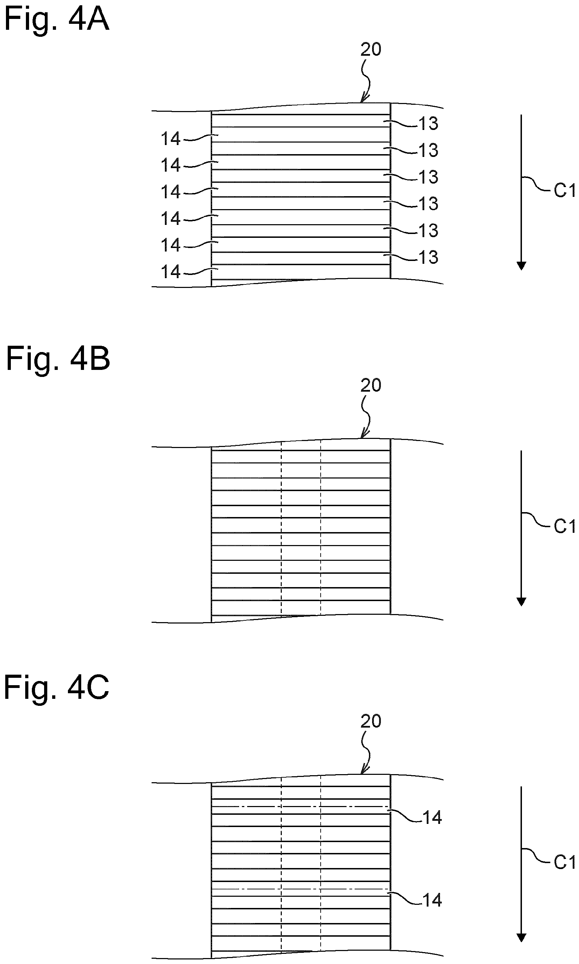

First, the apparatus 200 feeds the rolled cooling unit sheet 20. The cooling unit sheet 20 is wound in such a manner that the first direction in which the ridge portions 13 extend matches the width direction of the roll. Fig. 4A is a schematic plan view of the cooling unit sheet 20 as viewed from an arrow 4A illustrated in Fig. 3. In Fig. 4A, an arrow C1 indicates the conveyance direction of the cooling unit sheet 20. As illustrated in Fig. 4A, when the cooling unit sheet 20 is conveyed, the first direction in which the ridge portions 13 of the cooling unit sheet 20 extend is perpendicular to the conveyance direction C1. When the cooling unit sheet 20 is fed, the pair of first feed rollers 205 sandwiches the cooling unit sheet 20, and feeds the cooling unit sheet 20 in a predetermined direction according to the rotational direction of the first feed rollers 205. One of the pair of first feed rollers 205 can include a plurality of recessed portions and a plurality of protrusion portions corresponding to the ridge portions 13 and the valley portions 14 of the cooling unit sheet 20. This arrangement allows the first feed roller 205 to be engaged with the ridge portions 13 and the valley portions 14 of the cooling unit sheet 20, thereby being able to prevent the cooling unit sheet 20 from slipping on the first feed roller 205 even without the cooling unit sheet 20 securely sandwiched between the pair of first feed rollers 205.

The cooling unit sheet 20 fed by the first feed rollers 205 is conveyed while being attracted to the cutting drum 210. Subsequently, the cooling unit sheet 20 fed to the cutting drum 210 is cut. More specifically, the cooling unit sheet 20 conveyed by the cutting drum 210 is cut by a slit cutter 240 along the conveyance direction C1. Fig. 4B is a schematic plan view of the cooling unit sheet 20 immediately after it is cut by the slit cutter 240. In Fig. 4B, portions at which the cooling unit sheet 20 is cut by the slit cutter 240 are indicated by broken lines. As illustrated in Fig. 4B, the cooling unit sheet 20 is cut along the second direction. In the example illustrated in Fig. 4B, the cooling unit sheet 20 is cut along the second direction at two portions.

Further, the cooling unit sheet 20 is cut by a cutter 255. Fig. 4C is a schematic plan view of the cooling unit sheet 20 immediately after it is cut by the cutter 255. In Fig. 4C, portions at which the cooling unit sheet 20 is cut by the cutter 255 are indicated by long dashed short dashed lines. As illustrated in Fig. 4C, the cooling unit sheet 20 is cut along the first direction so as to be separated at predetermined intervals in the second direction. Preferably, the cutter 255 is provided on the second feed roller 250. The second feed roller 250 includes a plurality of recessed portions 250a and a plurality of protrusion portions 250b corresponding to the ridge portions 13 and the valley portions 14 of the cooling unit sheet 20, and further feeds the cooling unit sheet 20 attracted to the cutting drum 210. Preferably, the cutter 255 is provided on at least one of the plurality of protrusion portions 250b of the second feed roller 250. This arrangement allows the cooling unit sheet 20 to be cut at positions of the cooling unit sheet 20 that correspond to the valley portions 14 while the second feed roller 250 feeds the cooling unit sheet 20. The cooling unit sheet 20 is cut by the cutter 255 after being cut by the slit cutter 240 in the present embodiment, but the cutting order is not limited thereto. In other words, the cooling unit sheet 20 may be cut by the slit cutter 240 after being cut by the cutter 255.

A plurality of cooling unit sheets 20 is formed by cutting the cooling unit sheet 20 by the slit cutter 240 and the cutter 255 on the cutting drum 210. Preferably, the cutting drum 210 rotates at a higher speed than the circumferential speed of the first feed rollers 205, which feed the cooling unit sheet 20 to the cutting drum 210. This setting allows the cutting drum 210 to tension the cooling unit sheet 20 to prevent the cooling unit sheet 20 from being loosened, thereby assisting the cutting by the slit cutter 240 and the cutter 255. Further, the cutting drum 210 can slide relative to the cooling unit sheet 20 illustrated in Fig. 4B before the cooling unit sheet 20 is cut along the first direction, and create a space between the plurality of cooling unit sheets 20 illustrated in Fig. 4C after the cooling unit sheet 20 is cut along the first direction. This makes it easy for a drum at a subsequent stage (the acceleration drum 220 in the present embodiment) to attract each of the cooling unit sheets 20 separately as a result.

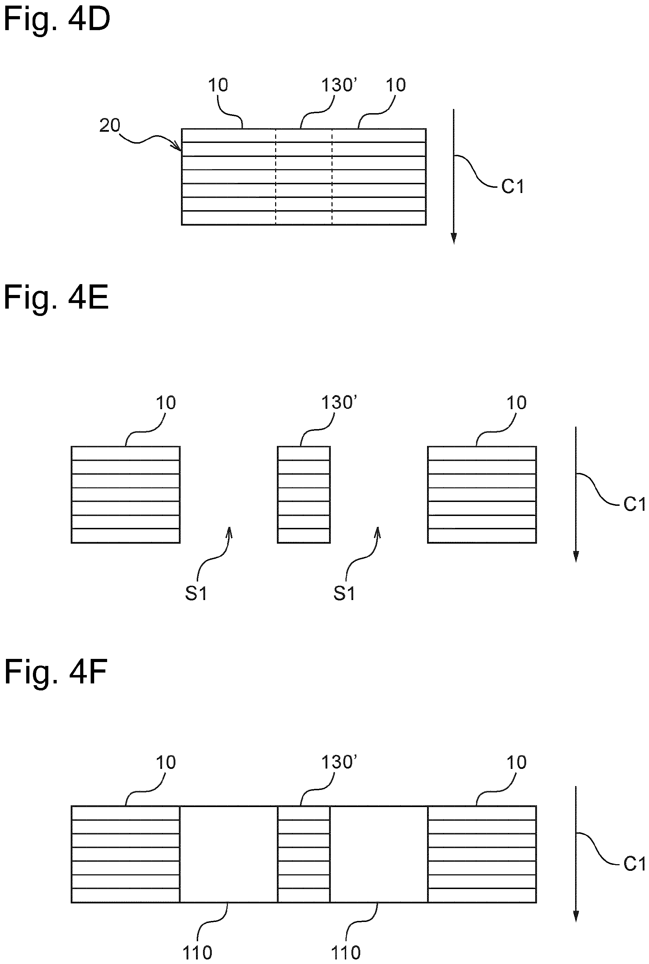

Fig. 4D is a schematic plan view of the cooling unit sheet 20 immediately after it is cut on the cutting drum 210. As illustrated in Fig. 4D, the cut cooling unit sheet 20 includes at least two cooling units 10 adjacent in the first direction. In the present embodiment, the cooling unit sheet 20 is cut along the second direction at two portions by the slit cutter 240, thereby including a pair of cooling units 10 located at the both ends in the first direction and a stopper material 130' located in the middle thereof. The stopper material 130' located in the middle is eventually cut into two pieces and forms two stoppers 130 configured as illustrated in Fig. 1B.

The cooling unit sheet 20 cut on the cutting drum 210 is attracted by an attraction unit 220a of the acceleration drum 220, and is conveyed to the separation drum 230. Preferably, the separation drum 230 rotates at a higher circumferential speed than the circumferential speed of the cutting drum 210. The attraction unit 220a of the acceleration drum 220 is movable in the circumferential direction of the acceleration drum 220. When receiving the cooling unit sheet 20 from the cutting drum 210, the attraction unit 220a rotates at a circumferential speed equal to the cutting drum 210. On the other hand, when transferring the cooling unit sheet 20 to the separation drum 230, the attraction unit 220a rotates at a circumferential speed equal to the circumferential speed of the separation drum 230. Due to that, the acceleration drum 220 can transfer the cooling unit sheet 20 to the separation drum 230 after increasing the space between the cooling unit sheets 20 in the conveyance direction C1. Preferably, a surface of the attraction unit 220a that attracts the cooling unit sheet 20 is formed into a recessed shape. This arrangement allows the attraction unit 220a to easily attract the cooling unit sheet 20 compared to a configuration that attracts the cooling unit sheet 20 on a flat surface.

The at least two cooling units 10 adjacent in the first direction that are formed from the cut cooling unit sheet 20 are spaced apart from each other in the first direction by the separation drum 230. Fig. 4E is a schematic plan view illustrating the two cooling units 10 spaced apart by the separation drum 230 and the stopper material 130'. As illustrated in Fig. 4E, the two cooling units 10 are spaced apart from the stopper material 130' away from each other, and a space S1 is formed between the stopper material 130' and each of the cooling units 10.

Subsequently, the separation drum 230 transfers the two cooling units 10 and the stopper material 130' illustrated in Fig. 4E to the conveyor 260, which conveys the flavor source 110. Two flavor sources 110 are disposed on the conveyor 260 in a direction perpendicular to the conveyance direction with a space created therebetween in correspondence with the stopper material 130' illustrated in Fig. 4E. Due to that, when the two cooling units 10 and the stopper material 130' are transferred onto the conveyor 260, the flavor sources 110 are disposed in the spaces S1 between the two cooling units 10 and the stopper material 130' illustrated in Fig. 4E, respectively.

Fig. 4F is a schematic plan view of the plurality of cooling units 10 and the stopper material 130' transferred to the conveyor 260. As illustrated, the flavor sources 110 are disposed between the two cooling units 10 and the stopper material 130', respectively. The two cooling units 10, the stopper material 130', and the two flavor sources 110 illustrated in Fig. 4F are wrapped in the single case sheet 125 illustrated in Fig. 1B in a process at a subsequent stage. After that, the single case sheet 125 containing them is cut at the central portion in the length direction (the horizontal direction) illustrated in Fig. 4F, and two cartridges 100 configured as illustrated in Fig. 1A are manufactured.

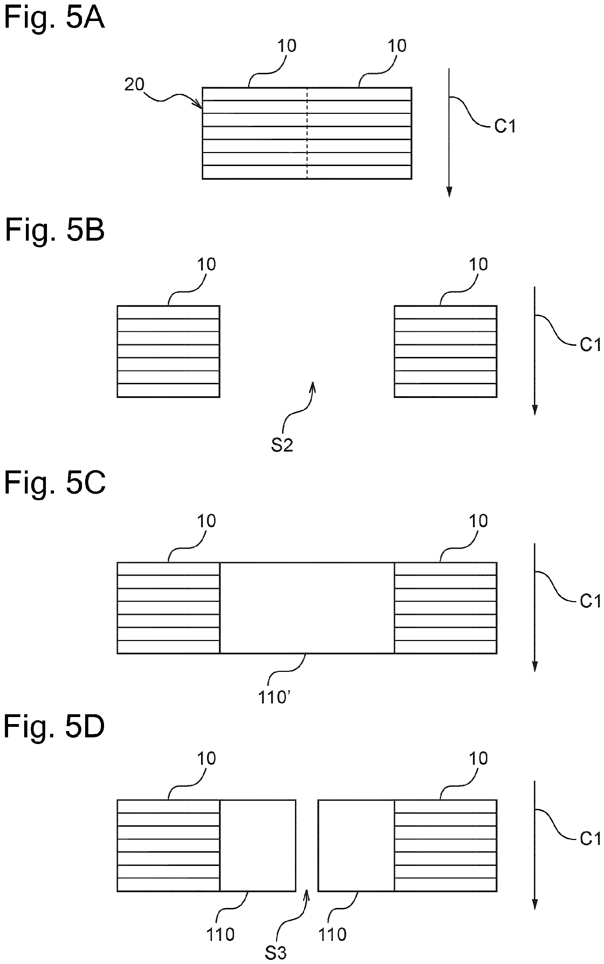

The cooling unit sheet 20 is cut along the second direction at two portions by the slit cutter 240 in the above-described method for manufacturing the cartridge 100, but is not limited thereto. The cooling unit sheet 20 may be cut along the second direction at one portion by the slit cutter 240. Fig. 5A illustrates a schematic plan view of the cooling unit sheet 20 manufactured by a manufacturing method according to another embodiment. In Fig. 5A, a portion at which the cooling unit sheet 20 is cut by the slit cutter 240 is indicated by a broken line. As illustrated, the cooling unit sheet 20 is cut along the second direction at one portion in the first direction. In the illustrated example, the cooling unit sheet 20 is cut along the second direction at the central portion in the first direction. As a result, two cooling units 10 adjacent in the first direction are formed.

Subsequently, in the cooling unit sheet 20 illustrated in Fig. 5A, the cooling units 10 are spaced apart from each other in the first direction on the separation drum 230. Fig. 5B is a schematic plan view illustrating the two cooling units 10 spaced apart by the separation drum 230. As illustrated in Fig. 5B, a space S2 is created between the two cooling units 10.

Subsequently, the separation drum 230 transfers the two cooling units 10 illustrated in Fig. 5B to the conveyor 260, which conveys the flavor source 110 illustrated in Fig. 3. A single flavor source 110' or two flavor sources 110 are disposed on the conveyor 260 at a position corresponding to the space S2 between the two cooling units 10 illustrated in Fig. 5B. As a result, when the two cooling units 10 are transferred to the conveyor 260, the single flavor source 110' or the two flavor sources 110 are disposed in the space S2 between the two cooling units 10 illustrated in Fig. 5B.

Fig. 5C is a schematic plan view of the two cooling units 10 transferred to the conveyor 260. As illustrated, the single flavor source 110' can be disposed between the two cooling units 10. The flavor source 110' can have, for example, approximately twice the length of the flavor source 110. The two cooling units 10 and the single flavor source 110' illustrated in Fig. 5C are wrapped in the single case sheet 125 illustrated in Fig. 1B in a process at a subsequent stage. After that, the single case sheet 125 containing them can be cut at the central portion in the length direction (the horizontal direction) illustrated in Fig. 5C. In this case, two cartridges 100 configured as illustrated in Fig. 1A with the stopper 130 omitted therefrom are manufactured.

Fig. 5D is a schematic plan view of the two cooling units 10 transferred to the conveyor 260. As illustrated, the two flavor sources 110 can be disposed between the two cooling units 10. A space S3 may be provided between the two flavor sources 110. The two cooling units 10 and the two flavor sources 110 illustrated in Fig. 5C are wrapped in the single case sheet 125 illustrated in Fig. 1B in a process at a subsequent stage. After that, the single case sheet 125 containing them is cut at the central portion in the length direction (the horizontal direction) illustrated in Fig. 5D, i.e., the space S3. In this case, two cartridges 100 configured as illustrated in Fig. 1A with the stopper 130 omitted therefrom are manufactured. Further, in the case of the example illustrated in Fig. 5D, the two flavor sources 110 cut so as to have equal lengths in advance can be disposed with the space S3 created therebetween. In this case, even if the cutting position in the length direction (the horizontal direction) illustrated in Fig. 5D is somewhat offset, the cutting at the space S3 allows the two flavor sources 110 to have even lengths. As a result, the two cartridges 100 can be prevented from having a difference in flavor taste due to the lengths of the flavor sources 110 in the two cartridges 100. In the case where the stopper 130 is omitted as illustrated in Fig. 5D, the flavor source 110 may be glued to the case 120.

Having described the embodiments of the present invention, the present invention shall not be limited to the above-described embodiments, and can be modified in various manners within the scope of the technical idea disclosed in the claims, specification, and drawings. Note that any shape and material not directly described or illustrated in the specification or drawings are still within the scope of the technical idea of the present invention insofar as they allow the present invention to achieve the actions and effects thereof.

Some of configurations disclosed in the present specification will be described below.

According to a first configuration, a method for manufacturing a flavor inhaler cartridge is provided. The flavor inhaler cartridge includes a flavor source configured to generate an aerosol by being heated, and a cooling unit including a corrugated portion having a plurality of ridge portions and a valley portion between the ridge portions. This manufacturing method includes feeding a cooling unit sheet used as a material of the cooling unit, cutting the cooling unit sheet along a second direction perpendicular to a first direction in which the plurality of ridge portions extends and cutting the cooling unit sheet along the first direction so as to separate the cooling unit sheet at a predetermined interval in the second direction, spacing at least two cooling units adjacent in the first direction, which are generated from the cut cooling unit sheet, apart from each other in the first direction, and disposing the flavor source between the at least two cooling units.

According to a second configuration, in the first configuration, the method for manufacturing the flavor inhaler cartridge includes feeding the cooling unit sheet to a cutting drum, and cutting the cooling unit sheet fed to the cutting drum.

According to a third configuration, in the second configuration, the method for manufacturing the flavor inhaler cartridge includes rotating the cutting drum at a higher speed than a circumferential speed of a feed roller configured to feed the cooling unit sheet to the cutting drum.

According to a fourth configuration, in the second or third configuration, the method for manufacturing the flavor inhaler cartridge includes cutting the cooling unit sheet along the first direction by a cutter provided on at least one of a plurality of protrusion portions of a feed roller while feeding the cooling unit sheet fed to the cutting drum by the feed roller. The feed roller includes a plurality of recessed portions and the plurality of protrusion portions corresponding to the ridge portions and the valley portion of the cooling unit sheet.

According to a fifth configuration, in any of the second to fourth configurations, the method for manufacturing the flavor inhaler cartridge includes spacing the at least two cooling units adjacent in the first direction, which are generated from the cut cooling unit sheet, apart from each other in the first direction by a separation drum, and rotating the separation drum at a circumferential speed higher than a circumferential speed of the cutting drum.

According to a sixth configuration, in any of the first to fifth configurations, the cooling unit includes a substrate sheet provided on a surface of the corrugated portion on one side. The manufacturing method includes feeding the rolled cooling unit sheet wound in such a manner that the corrugated portion faces outward and the substrate sheet faces inward.

According to a seventh configuration, in any of the first to sixth configurations, the method for manufacturing the flavor inhaler cartridge includes generating the two cooling units adjacent in the first direction and a stopper material located between the two cooling units by cutting the cooling unit sheet along the second direction, spacing the two cooling units adjacent in the first direction apart from the stopper material away from each other in the first direction, and disposing the flavor source between each of the two cooling units and the stopper material.

According to an eighth configuration, a cooling unit configured to be used in a flavor inhaler cartridge is provided. This cooling unit includes a corrugated portion including a plurality of ridge portions and a valley portion between the ridge portions, and a substrate sheet provided on a surface of the corrugated portion on one side.

### REFERENCE SIGNS LIST

- 10: cooling unit
- 12: corrugated portion
- 13: ridge portion
- 14: valley portion
- 17: substrate sheet
- 20: cooling unit sheet
- 100: cartridge
- 110, 110': flavor source
- 130: stopper
- 210: cutting drum
- 230: separation drum
- 250: second feed roller
- 255: cutter

## Claims

1. A method for manufacturing a flavor inhaler cartridge, the flavor inhaler cartridge including a flavor source configured to generate an aerosol by being heated and a cooling unit including a corrugated portion having a plurality of ridge portions and a valley portion between the ridge portions, the method comprising:
feeding a cooling unit sheet used as a material of the cooling unit;
cutting the cooling unit sheet along a second direction perpendicular to a first direction in which the plurality of ridge portions extends, and cutting the cooling unit sheet along the first direction so as to separate the cooling unit sheet at a predetermined interval in the second direction;
spacing at least two cooling units adjacent in the first direction, which are generated from the cut cooling unit sheet, apart from each other in the first direction; and
disposing the flavor source between the at least two cooling units.

2. The method for manufacturing the flavor inhaler cartridge according to claim 1, comprising:
feeding the cooling unit sheet to a cutting drum; and
cutting the cooling unit sheet fed to the cutting drum.

3. The method for manufacturing the flavor inhaler cartridge according to claim 2, comprising rotating the cutting drum at a higher speed than a circumferential speed of a feed roller configured to feed the cooling unit sheet to the cutting drum.

4. The method for manufacturing the flavor inhaler cartridge according to claim 2 or 3, comprising cutting the cooling unit sheet along the first direction by a cutter provided on at least one of a plurality of protrusion portions of a feed roller while feeding the cooling unit sheet fed to the cutting drum by the feed roller, the feed roller including a plurality of recessed portions and the plurality of protrusion portions corresponding to the ridge portions and the valley portion of the cooling unit sheet.

5. The method for manufacturing the flavor inhaler cartridge according to any one of claims 2 to 4, comprising:
spacing the at least two cooling units adjacent in the first direction, which are generated from the cut cooling unit sheet, apart from each other in the first direction by a separation drum; and
rotating the separation drum at a circumferential speed higher than a circumferential speed of the cutting drum.

6. The method for manufacturing the flavor inhaler cartridge according to any one of claims 1 to 5, wherein the cooling unit includes a substrate sheet provided on a surface of the corrugated portion on one side,
the manufacturing method comprising feeding the rolled cooling unit sheet wound in such a manner that the corrugated portion faces outward and the substrate sheet faces inward.

7. The method for manufacturing the flavor inhaler cartridge according to any one of claims 1 to 6, comprising:
generating the two cooling units adjacent in the first direction and a stopper material located between the two cooling units by cutting the cooling unit sheet along the second direction;
spacing the two cooling units adjacent in the first direction apart from the stopper material away from each other in the first direction; and
disposing the flavor source between each of the two cooling units and the stopper material.

8. A cooling unit configured to be used in a flavor inhaler cartridge, the cooling unit comprising:
a corrugated portion including a plurality of ridge portions and a valley portion between the ridge portions; and
a substrate sheet provided on a surface of the corrugated portion on one side.
